(19) Europäisches Patentamt
European Patent Office
Office européen des brevets

(11) Veröffentlichungsnummer : **0 112 289**
**B1**

# (12) EUROPÄISCHE PATENTSCHRIFT

(45) Veröffentlichungstag der Patentschrift : **09.09.87**

(51) Int. Cl.[4] : **A 01 N 25/32**, A 01 N 47/42

(21) Anmeldenummer : **83810555.9**

(22) Anmeldetag : **28.11.83**

## (54) N-Sulfonyl-imino-thiokohlensäure-diester als Herbizid-Antagonisten zum Schutz von Reiskulturen.

(30) Priorität : **03.12.82 CH 7048/82**

(43) Veröffentlichungstag der Anmeldung : **27.06.84 Patentblatt 84/26**

(45) Bekanntmachung des Hinweises auf die Patenterteilung : **09.09.87 Patentblatt 87/37**

(84) Benannte Vertragsstaaten : **AT BE CH DE FR GB IT LI LU NL SE**

(56) Entgegenhaltungen :
**EP-A- 0 010 058**
**EP-A- 0 011 473**
**DE-A- 2 644 446**

(73) Patentinhaber : **CIBA-GEIGY AG**
**Klybeckstrasse 141**
**CH-4002 Basel (CH)**

(72) Erfinder : **Töpfl, Werner, Dr.**
**Dorneckstrasse 68**
**CH-4143 Dornach (CH)**

**EP 0 112 289 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft N-Sulfonyl-imino-thiokohlensäure-diester, welche sich als Herbizid-Antagonisten zum Schützen von Reiskulturen vor durch Herbizide verursachte phytotoxische Schäden eignen. Dabei werden die N-Sulfonyl-imino-thiokohlensäure-diester gleichzeitig oder in kurzer Folge mit dem Herbizid den Kulturpflanzungen appliziert. Man kann auch ein Mittel anwenden, welches sowohl das Herbizid wie den N-Sulfonyl-imino-thiokohlensäure-diester enthält oder man kann die Samen mit dem N-Sulfonyl-imino-thiokohlensäure-diester vorbehandeln (beizen) und nachher die gesäte oder aufgelaufene Kultur mit dem Herbizid applizieren. Die Erfindung betrifft auch Mittel, welche die N-Sulfonyl-imino-thiokohlensäure-diester enthalten sowie ihre Verwendung.

Es ist bekannt, dass Herbizide aus den verschiedensten Stoffklassen, wie Triazine, Harnstoffderivate, Carbamate, Thiolcarbamate, Halogenacetanilide, Halogenphenoxyessigsäure usw. bei der Anwendung in wirksamer Dosis gelegentlich neben den zu bekämpfenden Unkräutern auch die Kulturpflanzen in gewissem Masse schädigen. Ueberdosen werden oft ungewollt und zufälligerweise appliziert, wenn sich Randzonen beim streifenweisen Spritzen überdecken, sei es durch Windeinwirkung oder durch falsches Einschätzen der Breitenwirkung des Spritzgerätes. Es können klimatische Verhältnisse oder eine Bodenbeschaffenheit vorliegen, so dass die für normale Bedingungen empfohlene Herbizidmenge als Ueberdosis wirkt. Die Qualität des Saatgutes kann bei der Herbizidverträglichkeit auch eine Rolle spielen. Um diesem Problem zu begegnen, sind schon verschiedene Stoffe vorgeschlagen worden, welche befähigt sind, die schädigende Wirkung des Herbizids auf die Reispflanze spezifisch zu antagonisieren, d. h. die Reiskultur zu schützen, ohne dabei die Herbizidwirkung auf die zu bekämpfenden Unkräuter merklich zu beeinflussen. Dabei hat es sich gezeigt, dass die vorgeschlagenen Gegenmittel sowohl bezüglich der Kulturpflanzen als auch bezüglich des Herbizids und gegebenenfalls auch in Abhängigkeit von der Applikationsart oft sehr artspezifisch wirken, d. h. ein bestimmtes Gegenmittel eignet sich oft nur für eine bestimmte Kulturpflanze und einige wenige herbizide Stoffklassen.

Die N-Sulfonyl-imino-thiokohlensäure-diester entsprechen der Formel I

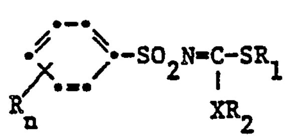

(I)

worin

n 0, 1, 2 oder 3,

R Halogen, Cyan, Nitro, einen Rest —Y—$C_1$-$C_4$-Alkyl, —Y—$C_3$-$C_6$ Alkenyl, —Y—$C_3$-$C_6$-Alkinyl, —Y-Phenyl oder —Y-Aralkyl, welcher unsubstituiert oder durch Halogen, Cyan, Nitro, $C_1$-$C_4$ Alkoxy, $C_1$-$C_4$ Alkoxycarbonyl, Carbamoyl, Sulfamoyl, $C_1$-$C_4$-Mono- oder Dialkylcarbamoyl, $C_1$-$C_4$-Mono- oder Dialkylsulfamoyl substituiert ist, ferner ein Rest —$COOR_3$, —$NR_3R_4$, —$CONR_3R_4$, —$NHCONR_3R_4$ oder —$SO_2NR_3R_4$ oder zwei benachbarte R bilden zusammen eine 3-4 gliedrige gesättigte oder ungesättigte Kohlenwasserstoffkette deren Kettenglieder einmal durch Sauerstoff oder Schwefel und/oder ein- bis dreimal durch Stickstoff ersetzt sein können.

$R_1$ und $R_2$ unabhängig voneinander je einen $C_1$-$C_4$ Alkyl-, $C_3$-$C_6$ Alkenyl-, $C_3$-$C_6$ Alkinyl-, Phenyl- oder Aralkylrest, der unsubstituiert oder durch Halogen, Cyan, Nitro, $C_1$-$C_4$ Alkyl, $C_1$-$C_4$ Halogenalkyl oder $C_1$-$C_4$ Alkoxy substituiert sein kann oder eines von

$R_1$ und $R_2$ auch Wasserstoff oder einen 5-6 gliedrigen, gesättigten oder ungesättigten Hererocyclus bedeuten, der über ein Kohlenstoffatom gebunden ist, 1-3 Heteroatome enthält und auch benzanneliert sein kann und der durch Halogen und/oder Methyl substituiert sein kann.

$R_3$ einen $C_1$-$C_4$ Alkyl-, $C_3$-$C_6$-Alkenyl-, $C_3$-$C_6$ Alkinyl-, $C_3$-$C_6$ Cycloalkylrest, welcher durch Halogen, Cyan, Nitro oder $C_1$-$C_4$ Alkyl substituiert sein kann,

$R_4$ Wasserstoff, dasselbe wie $R_3$, einen Phenyl- oder Aralkylrest, welcher durch Halogen, Cyan, Nitro, $C_1$-$C_4$ Alkyl, $C_1$-$C_4$ Halogenalkyl oder $C_1$-$C_4$ Alkoxy substituiert sein kann oder

$R_3$ und $R_4$ bilden zusammen mit dem Stickstoffatom, an das sie gebunden sind, einen 5-6 gliedrigen, gesättigten oder ungesättigten Heterocyclus, der noch Sauerstoff, Schwefel, Stickstoff oder einen Methyliminorest als Ringglied enthalten kann,

X Sauerstoff oder Schwefel und

Y die direkte Bindung oder eine Sauerstoff-, Schwefel-, Sulfinyl-, Sulfonyl-, Imino- oder Methyliminobrücke bedeuten.

Der Ausdruck Alkyl allein oder als Teil eines Substituenten umfasst verzweigte oder unverzweigte Alkylgruppen, welche die angegebene Anzahl Kohlenstoffatome enthalten. Beispiele sind Methyl, Aethyl, Propyl, Isopropyl, Butyl, Isobutyl, sec.-Butyl, tert.-Butyl. Entsprechend können die Alkenyl- und Alkinylgruppen geradkettig oder verzweigt sein.

Aralkylreste enthalten 1-4 Kohlenstoffe im Alkylteil während der Arylrest Naphthyl oder vorzugsweise Phenyl sein kann. Benzyl und Phenyläthyl sind die bevorzugten Aralkylreste.

Die Alkenyl- und Alkinylreste enthalten 3-6 Kohlenstoffatome, können verzweigt oder geradkettig sein

und eine oder zwei ungesättigte Stellen enthalten. Beispiele sind Allyl, Methallyl, Butenyl, Butadienyl, Pentenyl oder Hexenyl, Propargyl, Butin, Pentin und Hexin.

Als Cycloalkylreste kommen Cyclopropyl, Cyclobutyl, Cyclopentyl und Cyclohexyl in Frage. Heterocyclen können 5-6 gliedrig, gesättigt oder ungesättigt sein. Die durch $R_3$ und $R_4$ gebildeten Heterocyclen sind über Stickstoff gebunden, die anderen, von $R_1$ und $R_2$ gebildeten sind über Kohlenstoff gebunden. Beispiele sind Pyrrol, Pyrolidin, Imidazol, Pyrazol, Pyridin, Piperidin, Pyrimidin, Pyridazin, Oxazol, Oxazolin, Oxazolidin, Thiazol, Thiazolidin, Oxadiazol, Thiadiazol, Morpholin und Pyrazolringe, für $R_1$ und $R_2$ kommen auch Thiophen, Furan, Tetrahydrofuron, Pyran in Frage. Benzannellierte Heterocyclen welche zwei R mit dem Phenylring oder $R_1$ und $R_2$ bilden, sind z. B. Benzthiophen, Benzthiazol, Benzoxazol, Indol, Isoindol, Indazol, Isochinolin und Chinolin.

Als Halogen kommen vor allem Fluor, Chlor und Brom, gelegentlich auch Iod in Frage.

Gute Schutzwirkung wurde mit denjenigen N-Sulfonyl-imino-thiokohlensäureestern der Formel I erreicht, in denen

— X Schwefel bedeutet,

— X Schwefel und n 1 oder 2 bedeutet,

— X Schwefel oder Sauerstoff,

n 0, 1, 2 oder 3

R sich in der meta- oder para-Stellung zur Sulfonylgruppe befindet und Halogen, Cyan, $C_1$-$C_4$ Alkyl, $C_1$-$C_4$ Halogenalkyl, $C_1$-$C_4$ Alkoxy, $C_1$-$C_4$ Halogenalkoxy, $C_1$-$C_4$ Alkylthio, $C_1$-$C_4$ Alkylsulfonyl, Sulfamoyl, oder $C_1$-$C_4$ Dialkylsulfamoyl und $R_1$ und $R_2$ unabhängig voneinander je $C_1$-$C_4$ Alkyl, $C_2$-$C_5$ Alkoxyalkyl, $C_1$-$C_4$ Halogenalkyl, $C_3$-$C_6$ Alkenyl, $C_3$-$C_6$ Halogenalkenyl oder $C_3$-$C_6$ Alkinyl bedeuten ;

— X Schwefel

n 1 oder 2 bedeuten und R sich in der para- oder meta-Stellung zur Sulfonylgruppe befindet, während $R_1$ und $R_2$ die unter der Formel I gegebene Bedeutung haben ;

— X Schwefel

n 1 oder 2

R Halogen und gegebenenfalls —Y—$C_1$-$C_4$ Alkyl, Y—$C_1$-$C_4$ Halogenalkyl, Cyan oder Nitro.

Y die direkte Bindung oder Sauerstoff bedeuten, während $R_1$ und $R_2$ die unter der Formel I gegebene Bedeutung haben ;

— X Schwefel und $R_1$ und $R_2$ je Methyl bedeuten während R und n die unter der Formel I gegebene Bedeutung haben.

Speziell aufgefallen sind der N-(4-Chlorbenzolsulfonyl)-imino-dithiokohlensäure-dimethyl-ester der N-(4-Brombenzolsulfonyl)-imino-dithiokohlensäure-dimethyl-ester und der N-(3,4-Dichlor-benzolsulfonyl)-imino-dithiokohlensäure-dimethyl ester.

Die N-Sulfonylimino-thiokohlensäure-diester der Formel I eignen sich hervorragend, Reiskulturen vor der phytotoxischen Wirkung von Herbiziden verschiedenster Stoffklassen, wie Triazinen, Phenylharnstoffderivaten, Carbamaten, Thiolcarbamaten, Halogenacetaniliden, Halogenphenoxyessigsäureestern, substituierte Phenoxyphenoxyessigsäureestern und -propionsäureestern, substituierten Pyridinoxyphenoxyessigsäureestern und -propionsäureestern, Benzoesäurederivaten usw. zu schützen, sofern diese nicht oder nicht genügend selektiv wirken, also neben den zu bekämpfenden Unkräutern auch den Reis mehr oder weniger schädigen. Die Erfindung betrifft auch Mittel, welche die N-Sulfonyl-imino-thiokohlensäure-diester der Formel I zusammen mit Herbiziden enthalten.

Als Gegenmittel oder Antidoten sind schon verschiedene Stoffe vorgeschlagen worden, welche befähigt sind, die schädigende Wirkung eines Herbizides auf die Kulturpflanze spezifisch zu antagonisieren, d. h. die Kulturpflanze zu schützen, ohne dabei die Herbizidwirkung auf die zu bekämpfenden Unkräuter merklich zu beeinflussen. Dabei kann ein ein solches Gegenmittel, auf Safener genannt, je nach seinen Eigenschaften, zur Vorbehandlung des Saatgutes der Kulturpflanze (Beizung des Samens oder der Stecklinge) oder vor der Saat in die Saatfurchen oder als Tankmischung zusammen mit dem Herbizid vor oder nach dem Auflaufen der Pflanzen verwendet werden.

So beschreibt die GB-A-1 277 557 die Behandlung von Samen bzw. Sprösslingen von Weizen und Hirse mit gewissen Oxamsäureestern und Amiden vor dem Angriff durch N-Methoxymethyl-2',6'-diäthyl-chloracetanilid (Alachlor). In anderen Literaturstellen (DE-A-1 952 910, DE-A-2 245 471, FR-A-2 021 611) werden Gegenmittel zur Behandlung von Getreide, Mais- und Reis-Samen zum Schutz gegen den Angriff herbizider Thiolcarbamate vorgeschlagen. In der DE-A-1 567 075 und der UP-A-3 131 509 werden Hydroxy-amino-acetanilide und Hydantoine für den Schütz von Getreidesamen gegenüber Carbamaten wie IPC, CIPC vorgeschlagen. In der DE-A-2 644 446 werden N-Phenylsulfonylthiocarbamate beschrieben, welche als Gegenmittel Feldfrüchte vor der phytotoxischen Wirkung von Thiolcarbamat-Herbiziden schützen. In der US-A-4 317 310 werden 2-Imino-1,3-thiolane, -1,3-dithiole, -1,3-dithiane, -1,3-dithiethane und -1,3-oxathiole zum Schutz von Reiskulturen vor der Wirkung von Chloracetanilid- und Thiolcarbamat-Herbiziden empfohlen. In der weiteren Entwicklung haben sich alle diese Präparate jedoch als ungenügend erwiesen.

Ueberraschenderweise besitzen die N-Sulfonyl-imino-thiokohlensäure-diester der Formel I die Eigenschaft, Reispflanzen vor der phytotoxischen Wirkung von Herbiziden der verschiedensten Stoffklassen wie beispielsweise Chloracetanilide, Chloracetamide, Carbamate und Thiocarbamate, Diphenyläther und Nitrodiphenyläther, Benzoesäurederivate, Triazine und Triazinone, Phenylharnstoffe, Nitroaniline,

Oxdiazolone, Pyridyloxyphenoxyderivate, Phosphate und Pyrazole zu schützen, sofern diese nicht oder ungenügend tolerant sind.

Vorzugsweise wird die Reispflanze vor Herbiziden der Klassen Chloracetanilide, Chloracetamide, Thiocarbamate, Phosphate durch die erfindungsgemässen N-Sulfonyl-imino-thiokohlensäure-diester geschützt.

Ein solches Gegenmittel oder Antidote der Formel I kann je nach Anwendungszweck zur Vorbehandlung des Saatgutes der Kulturpflanze (Beizung der Samen) eingesetzt oder vor oder nach der Saat in den Erdboden gegeben werden oder aber für sich allein oder zusammen mit dem Herbizid vor oder nach dem Auflaufen der Pflanzen appliziert werden. Die Behandlung der Pflanze oder des Saatgutes mit dem Antidote kann daher grundsätzlich unabhängig vom Zeitpunkt der Applikation der phytotoxischen Chemikalie erfolgen. Sie kann jedoch auch gleichzeitig durchgeführt werden (Tankmischung). Vorauflauf-Behandlung schliesst sowohl die Behandlung der Anbaufläche vor der Aussaat (ppi = « pre plant incorporation ») als auch die Behandlung der angesäten, aber noch nicht bewachsenen Anbauflächen ein.

Die Aufwandmengen des Antidotes im Verhältnis zum Herbizid richten sich weitgehend nach der Anwendungsart. Sofern eine Feldbehandlung vorgenommen wird, entweder als Tankmischung oder bei getrennter Applikation von Herbizid und Gegenmittel, verhalten sich die Mengen von Gegenmittel zu Herbizid in der Regel 1 : 100 bis 10 : 1, bevorzugt wird jedoch der Bereich 1 : 5 bis 8 : 1, besonders 1 : 1.

Bei Samenbeizung und ähnlich gezielten Schutzmassnahmen werden jedoch weit geringere Mengen Gegenmittel im Vergleich mit den z. B. später pro Hektar Anbaufläche verwendeten Herbizidmengen benötigt. Bei der Samenbeizung werden üblicherweise pro kg Samen 0,1-10 g Gegenmittel benötigt, die bevorzugte Menge liegt zwischen 1 und 2 Gramm. Falls das Gegenmittel kurz vor der Aussaat durch Samenquellung appliziert werden soll, werden z. B. Gegenmittel-Lösungen, welche den Wirkstoff in einer Konzentration von 1-10 000 ppm enthalten, bevorzugt 100-1 000 ppm verwendet.

In der Regel folgen sich protektive Massnahmen wie Samenbeizung mit einem Gegenmittel der Formel I und mögliche spätere Feldbehandlung mit Agrarchemikalien in zeitlich grösserem Abstand. Vorbehandeltes Saat- und Pflanzengut kann später in Landwirtschaft, Gartenbau und Fortstwirtschaft mit unterschiedlichen Chemikalien in Berührung kommen.

Die Erfindung bezieht sich daher auch auf den Reis-schützende Mittel, die als Wirkstoff ein Gegenmittel der Formel I zusammen mit üblichen Trägerstoffen enthalten. Solche Mittel können gegebenenfalls zusätzlich mit jenen Herbizid gemischt sein, vor deren Einfluss die Kulturpflanze geschützt werden soll.

Das Gegenmittel soll überall dort eingesetzt werden, wo eine Kulturpflanze vor der Phytotoxizität einer Chemikalie geschützt werden soll. Als Herbizide, vor deren Wirkung es die Kulturpflanzen zu schützen gilt, seien beispielsweise folgende genannt :

Chloracetanilide : 2-Chlor-2',6'-diäthyl-N-(2''-propyloxyäthyl) acetanilid (« Pretilachlor »), 2-Chlor-6'-äthyl-N-(2''-methoxy-1''-methyläthyl)-acet-o-toluidid (« Metolachlor »), 2-Chlor-2',6'-diäthyl-N-(butoxymethyl) acetanilid (« Butachlor »), 2-Chlor-6'-äthyl-N-(äthoxymethyl) acet-o-toluidid (« Acetochlor »), 2-Chlor-6'-äthyl-N-(2''-propoxy-1''-methyläthyl) acet-o-toluidid, 2-Chlor-2',6'-dimethyl-N-(2''-methoxy-1''-methyläthyl) acetanilid, 2-Chlor-2',6-dimethyl-N(2''-methoxyäthyl) acetanilid (« Dimethachlor »), 2-Chlor-2',6'-diäthyl-N-(pyrazol-1-ylmethyl) acetanilid, 2-Chlor-6'-äthyl-N-(pyrazol-1-ylmethyl) acet-o-toluidid, 2-Chlor-6'-äthyl-N-(3,5-dimethyl-pyrazol-1-ylmethyl) acet-o-toluidid, 2-Chlor-6'-äthyl-N-(2''-butoxy-1''-methyläthyl) acet-o-toluidid (« Metazolachlor »), 2-Chlor-6'-äthyl-N-(2''-butoxyl-1''-(methyläthyl) acet-o-toluidid, 2-Chlor-2'-trimethylsilyl-N-(butoxymethyl)-acetanilid, 2-Chlor-2',6'-diäthyl-N-(methoxymethyl) acetanilid (« Alachlor ») und 2-Chlor-2',6'-diäthyl-N-(äthoxycarbonylmethyl) acetanilid.

Chloracetamide : N-[1-Isopropyl-2-methylpropen-1-yl(1)]-N-(2'-methoxyäthyl)-chloracetamid.

Sulfonylharnstoffe : N-(2-Chlorbenzolsulfonyl)-N'-(4-methoxy-6-methyl-1,3,5-triazinyl) harnstoff (DPX 4189). N-(2-Chloräthylbenzolsulfonyl)-N-(4-methoxy-6-methyl-1,3,5-triazinyl) harnstoff, N-(2-Methoxyäthylbenzolsulfonyl)-N'-(4-methoxy-6-methyl-1,3,5-triazinyl) harnstoff.

Carbamate und Thiocarbamate : N-(3',4'-Dichlorphenyl)-propionanilid (« propanil »), S-4-Chlorbenzyl-diäthyl-thiocarbamat (« Thiobencarb »), S-Aethyl-N,N-hexamethylen-thiocarbamat (« Molinate »), S-Aethyl-dipropylthiocarbamat (« EPIC »), N,N-di-sec.Butyl-S-benzyl-thiocarbamat (Drepamon), S-(2,3-Dichlorallyl)-di-isopropylthiocarbamat und S(2,3,3-Trichlorallyl)-di-isopropylthiocarbamat (« Di- und Tri-allate »), 1-(Propylthiocarbonyl)-decahydro-chinaldin, S-4-Benzyldiäthylthiocarbamat sowie entsprechende Sulfinylcarbamate.

Diphenyläther und Nitrodiphenyläther : 2,4-Dichlorphenyl-4'-nitrophenyläther (« Nitrofen »), 2-Chlor-1-(3'-äthoxy-4'-nitrophenoxy)-4-trifluormethyl-benzol (« Oxyfluorfen », 2',4'-Dichlorphenyl-3-methoxy-4-nitrophenyl-äther (« Chlormethoxinyl »), 2-[4'-(2'',4''-Dichlorphenoxy)-phenoxy] propionsäure-methylester, N-(2'-Methoxyäthyl)-2-[5'-(2''-chlor-4''-trifluormethylphenoxy)-phenoxy]-propionsäureamid.

Benzoesäurederivate: Methyl-5-(2',4'-dichlorphenoxy)-2-nitrobenzoat (« Bifenox »), 5-(2'-Chlor-4'-trifluormethylphenoxy)-2-nitrobenzoesäure (« Acifluorfen »), 2,6-Dichlorbenzonitril (« Dichlobenil »).

Triazine und Triazinone: 2,4-Bis(isopropylamino)-6-methylthio-1,3,5-triazin (« Prometryn », 2,4-bis(äthylamino)-6-methylthio-1,3,5-triazin (« Simetryn »), 2-(1',2'-Dimethylpropylamino)-4-äthylamino-6-methylthio-1,3,5-triazin (« Dimethametryn »), 4-Amino-6-tert.butyl-4,5-dihydro-3-methylthio-1,2,4-triazin-5-on (« Metribuzin »).

Phenylharnstoffe: N-(3'-Isopropylphenyl)-N',N'-dimethyl-harnstoff (« Isoproturon «), N-(3',4'-Dimethylbenzyl)-N'-4-tolyl-harnstoff (« Dimuron »), N-(3'-Chlor-4'-isopropylphenyl)-N',N'-(3-methyl-pentamethylen-1,5-yl)-harnstoff.

Nitroaniline: 2,6-Dinitro-N,N-dipropyl-4-trifluormethylanilin (« Trifluralin »), N-(1'-Aethylpropyl)-2,6-dinitro-3,4-xylidin (« Pendimethalin »).

Oxadiazolone: 5-tert.-Butyl-3-(2'-4'-dichlor-5'-isopropoxyphenyl)-1,3,4-oxadiazol-2-on (« Oxdiazon »).

Pyridyloxyphenoxyderivate: 2-[4'-(3",5"-Dichlorpyridyl-2"-oxy) phenoxy]-propionsäure-(2-propinyl) ester.

Phosphate: S-2-Methylpiperidino-carbonylmethyl-O,O-dipropyl-phosphorodithioat (« Piperophos »).

Pyrazole: 1,3-Dimethyl-4-(2',4'-dichlorbenzoyl)-5-(4'-tolylsulfonyloxy)-pyrazol.

Ein N-Sulfonyl-imino-thiokohlensäure-diester der Formel I oder ein Mittel, welches dieses Gegenmittel enthält, kann wahlweise vor oder nach der Applikation des Herbizides oder auch gleichzeitig mit diesem angewendet werden. Besonders rationell hat sich das Behandeln der Samen durch eine das Gegenmittel enthaltende Lösung (Samenbeizung) erwiesen. Dabei kann das Lösungsmittel verdunstet werden und der Samen trocken, mit einem Gegenmittelbelag behaftet zur Anwendung kommen oder man kann der Samen in einer wässerigen, das Gegenmittel enthaltenden Lösung vorquellen und in diesem Zustand aussäen, wie es beispielsweise bei Reis üblich ist.

Die N-Sulfonyl-imino-thiokohlensäure-diester der Formel I werden hergestellt, indem man in einem aprotischen dipolaren Lösungsmittel ein Benzolsulfonamid der Formel II

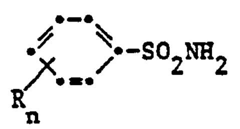

(II)

worin R und n die oben gegebene Bedeutung haben, in Gegenwart einer einer Base mit Schwefelkohlenstoff umsetzt und das entstandene N-Sulfonyl-imino-kohlensäuresalz der Formel III

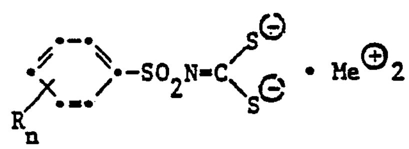

(III)

worin R, n die oben gegebene Bedeutung haben und Me für ein Alkalimetallion steht unverzüglich mit einem Halid oder Sulfat der Formel IV weiter umsetzt

$$R_1\text{—Hal},\ R_1SO_4Y \qquad (IV),$$

worin $R_1$ die gegebene Bedeutung hat und Hal ein Halogenatom, vorzugsweise Chlor, Brom oder Iod und Y Methyl oder p-Tolyl ist.

Dabei entsteht, je nach der aufgewendeten Menge und nach ansäuern der N-Sulfonyl-imino-mono- oder dithiokohlensäureester der Formel Ia oder Ib

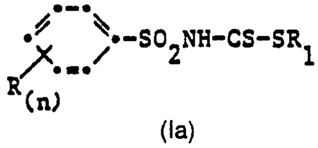

oder

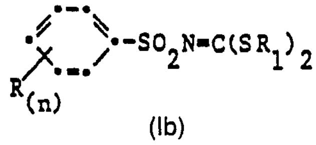

(Ia) (Ib)

worin R, $R_1$ und n die oben gegebene Bedeutung haben. Während der Dithiokohlensäure-diester der Formel Ib bereits Endprodukt ist, wird der Dithiokohlensäure-monoester der Formel Ia mit einem Alkohol der Formel V

$$R_2—OH \qquad (V)$$

umgesetzt, zum Thiokohlensäure-O-ester der Formel VI

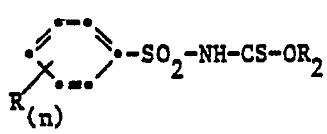

(VI)

worin R, $R_2$ und n die oben gegebene Bedeutung haben.

Dieser Thiokohlensäure-O-ester wird anschliessend in Gegenwart einer Base mit einem Halid oder Sulfat der oben gegebene Formel IV behandelt, worauf man einen gemischten Dithiokohlensäure-O,S-ester der Formel Ic erhält

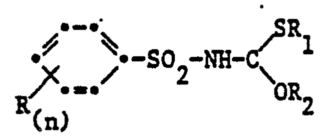

(Ic)

Diese Reaktionen sind bekannt und beispielsweise in Chem. Ber. 99 (1966) 2885-2899 beschrieben.

Als aprotische dipolare Lösungsmittel für die Umsetzung des Sulfonamides mit Schwefelkohlenstoff kommen beispielsweise Dimethylformamid, Dimethylsulfoxid, N-Methylpyrrolidon und Acetonitril in Betracht, in deren Lösung das entstandene Salz vorzugsweise gleich weiterverarbeitet wird.

Die Umsetzungen werden bei Raumtemperatur oder unter Kühlung durchgeführt da oft exotherme Reaktionen auftreten welche das Gemisch auf 50-60 °C erwärmten.

Die N-Sulfonyl-imino-thiokohlensäure-diester der Formel I können für sich allein oder zusammen mit den zu antagonisierenden Herbiziden verwendet werden.

Dabei werden Verbindungen der Formel I in unveränderter Form oder vorzugsweise zusammen mit den in der Formulierungstechnik üblichen Hilfsmitteln eingesetzt und werden daher z. B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z. B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d. h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen : Aromatische Kohlenwasserstoffe, bevorzugt die Fraktionen $C_8$ bis $C_{12}$, wie z. B. Xylolgemische oder substituierte Naphthaline, Phthalsäureeester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder äthyläther, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl ; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie z. B. Bimsstein, Ziegelbruch, Sepiolit oder Bentonit, als nicht sorptive Trägermaterialien z. B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen eignen sich die Alkali-, Erdalkali- oder gegebenenfalls substituierten Ammoniumsalze von

höheren Fettsäuren ($C_{10}$-$C_{22}$), wie z. B. die Na- oder K-Salze der Oel- oder Stearinsäure, oder von natürlichen Fettsäuregemischen, die z. B. aus Kokosnuss- oder Talgöl gewonnen werden können. Ferner sind auch die Fettsäure-methyl-taurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit 8-22 C-Atomen. Alkylarylsulfonate sind z. B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate, wie z. B. Salze des Phosphorsäureesters eines p-Nonylphenol-(4-14)-Aethylenoxid-Adduktes oder Phospholipide in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinussölpolyglykoläther, Polypropylen-Polyäthylenoxyaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitantrioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z. B. das Stearyltrimethylammoniumchlorid oder das Benzyldi(2-chloräthyl)äthylammoniumbromid.

Die in der Formulierungstechnik gebräuchlichen Tenside sind u. a. in folgenden Publikationen beschrieben :

« Mc Cutcheon's Detergents and Emulsifiers Annual » MC Publishing Corp., Ringwood, New Jersey, 1979.

J. and M. Ash, « Encyclopedia of Surfactants » Vol. I-III », Chemical Publishing Co., Inc. New York, 1980-81.

H. Stache « Tensid Taschenbuch » 2. Auflage C. Hanser Verlag, München und Wien, 1981.

Die herbiziden Zubereitungen enthalten in der Regel 0,1 bis 99 %, insbesondere 0,1 bis 95 %, Wirkstoffe der Formel I, 1 bis 99 % eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel, sowie Dünger oder andere Wirkstoffe zur Erzielung spezieller Effekte enthalten.

In den nachfolgenden Beispielen sind die Temperaturen in Celsiusgraden angegeben, Prozente und Angaben von « Teilen » beziehen sich auf das Gewicht.

Beispiel 1 : Herstellung von N-(4-Chlorbenzolsulfonyl)-imino-thiokohlensäure-O,S-dimethylester.

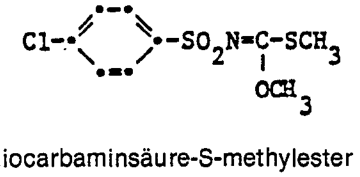

a) N-4-(Chlorbenzolsulfonyl)-dithiocarbaminsäure-S-methylester

384 g 4-Chlorbenzolsulfonamid (2 Mol) werden in 750 ml Dimethylformamid gelöst. Dann tropft man innerhalb von 4 Studen gleichzeitig unter Rühren 320 g 50 %ige wässrige Natronlauge (8 Mol) und 152 g

Schwefelkohlenstoff dazu. Die Temperatur wird durch Kühlen auf 10-15 °C gehalten. Die entstandene tiefrote Lösung des Dinatrium-Salzes wird während 2 Stunden bei Raumtemperatur nachgerührt. Dann tropft man 254 g Dimethylsulfat (2 Mol) dazu wobei die Temperatur durch Kühlen auf 15 °C gehalten wird. Der Ansatz wird 12 Stunden bei Raumtemperatur nachgerührt und dann mit Wasser auf 6 Liter verdünnt. Die trübe gelbe Lösung wird über Celite filtriert und dann mit konzentrierter Salzsäure sauer gestellt. Der N-(4-Chlorbenzolsulfonyl)-dithiocarbaminsäure-S-methylester fallt dabei als Oel aus und kristallisiert nach kurzer Zeit. Nach dem Abkühlen und Waschen mit Wasser wird die Verbindung mit wenig kaltem Methanol verrührt und abgekühlt. Man erhält so 270 g (48 % der Theorie) des obigen Esters als farbloses Kristallpulver. Schmelzpunkt 135-139 °C (Zers.).

b) N-(4-Chlorbenzolsulfonyl)-thiocarbaminsäure-O-methylester

14,1 g N-(4-Chlorphenylsulfonyl)-dithiocarbaminsäure-S-methylester (0,05 Mol) werden in 100 ml Methanol unter Rückfluss gekocht. Nach ca. 5 Stunden war die Mercaptanentwicklung beendet. Das Lösungsmittel wird am Rotationsverdampfer abdestilliert und der Rückstand aus Methanol umkristallisiert. Ausbeute 11,6 g (87 % der Theorie) Schmelzpunkt 129-130 °C.

c) N-(4-Chlorbenzolsulfonyl)-imino-thiokohlensäure-O,S-dimethylester

6,65 g N-(4-Chlorbenzolsulfonyl)-thiocarbaminsäure-S-methylester (0,025 Mol) werden in 250 ml Dimethylformamid gelöst und dazu gibt man 2 g 50 %ige Natronlauge (0,025 Mol). Unter Kühlung und Rühren werden danach 3,15 g Dimethylsulfat (0,025 Mol) zugetropft und 2 Stunden bei 30 °C nachgerührt. Der Ansatz wird dann in 300 ml Eiswasser eingerührt und der Niederschlag abfiltriert. Nach Umkristallisieren aus Methanol erhält man 5,3 g (75 % der Theorie) des obigen gemischten Esters. Schmelzpunkt 96-97 °C.

Beispiel 2 : Herstellung von N-(4-Chlorbenzolsulfonyl)-imino-dithiokohlensäure-S,S-dimethylester

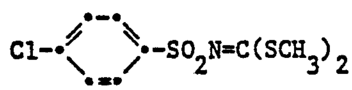

767 g 4-Chlorbenzolsulfonamid (4 Mol) werden in 1 500 ml Dimethylformamid gelöst. Dazu gibt man unter Rühren und Kühlen, so dass die Temperatur zwischen 10 und 15 °C bleibt, während 5 Stunden gleichzeitig tropfenweise 640 g 50 %ige wässrige Natronlauge (8 Mol) und 305 g Schwefelkohlenstoff (4 Mol). Die entstandene tiefrote Lösung des Dinatrium-Salzes wird noch 2 Stunden bei Raumtemperatur nachgerührt. Dann werden 1 008 g Dimethylsulfat dazu getropft. Die Temperatur wird dabei durch Kühlen auf 15 °C gehälten. Die entstandene Dispersion wird dann 2 Stunden bei 35 °C nachgerührt und anschliessend mit 5 Liter Wasser verdünnt. Es fällt ein Niederschlag aus, der abfiltriert wird. Nach Umkristallisieren aus Methanol erhält man so 820 g (69 % der Theorie) N-(4-Chlorbenzolsulfonyl)-imino-dithiokohlensäure-S,S-dimethylester als farblose Kristallpulver. Schmelzpunkt 93-94 °C.

Analog zu diesen Beispielen werden folgende Verbindungen hergestellt :

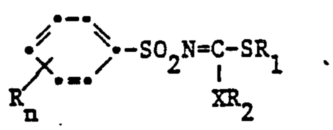

| Verb. Nr. | $R_n$ | $R_1$ | X | $R_2$ | phys. Daten |
|---|---|---|---|---|---|
| 1 | H | $CH_3$ | S | $CH_3$ | Smp. 106-108° |
| 2 | H | $C_2H_5$ | S | $C_2H_5$ | Smp. 90° |
| 3 | 4-$CH_3$ | $CH_3$ | S | $CH_3$ | Smp. 108-110° |
| 4 | 4-$CH_3$ | $C_2H_5$ | S | $C_2H_5$ | Smp. 79° |
| 5 | 4-Cl | $CH_3$ | S | $CH_3$ | Smp. 94-95° Beisp.2 |
| 6 | 4-Cl | $C_2H_5$ | S | $C_2H_5$ | Smp. 51° |

(Fortsetzung)

| Verb. Nr. | $R_n$ | $R_1$ | X | $R_2$ | phys. Daten |
|---|---|---|---|---|---|
| 7 | 4-Br | $CH_3$ | S | $CH_3$ | Smp. 109-112° |
| 8 | 4-Br | $C_2H_5$ | S | $C_2H_5$ | Smp. 66-68° |
| 9 | 4-$CH_3$ | $C_2H_5$ | S | $CH_2CH=CH_2$ | Smp. 61-64° |
| 10 | 4-$CH_3$ | $CH_3$ | S | $CH_2C\equiv CH$ | Smp. 113-115° |
| 11 | 4-$CF_3$ | $CH_3$ | S | $CH_3$ | |
| 12 | 4-$OCF_3$ | $CH_3$ | S | $CH_3$ | |
| 13 | 4-Cl | $CH_2$-CH=$CH_2$ | S | $CH_2$-CH=$CH_2$ | |
| 14 | 3,4 $Br_2$ | $CH_3$ | S | $CH_3$ | |
| 15 | 3,4 $Cl_2$ | $CH_3$ | S | $CH_3$ | |
| 16 | 3-Br, 4-Cl | $CH_3$ | S | $CH_3$ | |
| 17 | 4-Cl | $CH_3$ | O | $CH_3$ | Smp. 96-97 Beisp.1 |
| 18 | 4-Cl | $C_2H_4OCH_3$ | S | $C_2H_4OCH_3$ | |
| 19 | 4-$SCH_3$ | $CH_3$ | S | $CH_3$ | |
| 20 | 4-$OC_3H_7$-i | $CH_3$ | S | $CH_3$ | |
| 21 | 4-$OCHF_2$ | $CH_3$ | S | $CH_3$ | |
| 22 | 3-$CH_3$ | $CH_3$ | S | $CH_3$ | |
| 23 | 3-Cl | $CH_3$ | S | $CH_3$ | Smp. 83-85 |
| 24 | 3-F | $CH_3$ | S | $CH_3$ | |
| 25 | 4-F | $CH_3$ | S | $CH_3$ | Smp. 112-114 |
| 26 | 3-$CF_3$ | $CH_3$ | S | $CH_3$ | Smp. 53-56 |
| 27 | 4-Cl | $C_2H_5$ | O | $C_2H_5$ | |
| 28 | 4-Br | $CH_3$ | O | $CH_3$ | |
| 29 | 4-Br | $C_2H_5$ | O | $C_2H_5$ | |
| 30 | 4-$OCF_3$ | $CH_3$ | O | $CH_3$ | |
| 31 | 4-$CF_3$ | $CH_3$ | O | $CH_3$ | |
| 32 | 3,4 $Cl_2$ | $CH_3$ | O | $CH_3$ | |
| 33 | 3,4 $Br_2$ | $CH_3$ | O | $CH_3$ | |
| 34 | 4-Cl | $C_2H_4OCH_3$ | O | $CH_3$ | |
| 35 | 4-Cl | $CH_2CH=CH_2$ | O | $CH_3$ | Oel |
| 38 | 3-$NO_2$ | $CH_3$ | S | $CH_3$ | Smp. 130-132 |
| 39 | 3,4 $Cl_2$ | $CH_3$ | S | $CH_3$ | Smp. 122-124 |
| 41 | 4-Cl | $CH_3$ | O | $C_3H_7$iso | Smp. 53-56 |

(Fortsetzung)

| Verb. Nr. | $R_n$ | $R_1$ | X | $R_2$ | phys. Daten Smp. °C |
|---|---|---|---|---|---|
| 43 | 4-Cl | $CH_3$ | S | benzyl | 95-98 |
| 44 | 4-Cl | phenyl | O | $CH_3$ | 75-38 |
| 45 | 4-Cl | $CH_2C{\equiv}CH$ | O | $CH_3$ | 91-95 |
| 46 | 4-Cl | $CH_3$ | O | phenyl | 127-129 |
| 47 | 4-Cl | $CH_3$ | S | phenyl | 130-132 |
| 48 | 4-Cl | $CH_3$ | S | H | 139-141 |
| 49 | 4-Br | 4-fluorobenzyl | S | 4-fluorobenzyl | 120-125 |
| 50 | 4-Br | $CH_3$ | S | 4-fluorobenzyl | 108-110 |
| 51 | 4-Br | $CH_3$ | S | H | 135-137 |
| 52 | 4-Cl | 4-chlorbenzyl | S | 4-chlorbenzyl | 123-125 |
| 54 | 4-Br, 3-Cl | $CH_3$ | S | $CH_3$ | 115-121 |
| 55 | 4-Br, 3-$CH_3$ | $CH_3$ | S | $CH_3$ | 110-112 |
| 56 | 3-Cl, 4-$CF_3$ | $CH_3$ | S | $CH_3$ | 113-115 |
| 57 | 3,4 $Br_2$ | $CH_3$ | S | $CH_3$ | 104-106 |
| 58 | 4-$COOCH_3$ | $CH_3$ | S | $CH_3$ | |
| 59 | 4-$COOC_2H_5$ | $CH_3$ | S | $CH_3$ | |
| 60 | 4-COOH | $CH_3$ | S | $CH_3$ | |
| 61 | 3,4 $F_2$ | $CH_3$ | S | $CH_3$ | |
| 62 | 4-Cl | $CH_3$ | S | $CH_3$ | 94-95 |
| 63 | 4-i$C_3H_7$ | $CH_3$ | S | $CH_3$ | 97-99 |
| 64 | 4-Cl,2,5$(OCH_3)_2$ | $CH_3$ | S | $CH_3$ | 169-171 |
| 65 | 4-$(C_2H_5OCO)_2C{=}CHNH-$ | $CH_3$ | S | $CH_3$ | 138-142 |
| 66 | 2-$CH_3$, 4-Cl | $CH_3$ | S | $CH_3$ | 121-123 |
| 67 | 2,3-$OC_2H_4$ | $CH_3$ | S | $CH_3$ | 161-163 |
| 68 | 2-$CH_3$, 3-Cl | $CH_3$ | S | $CH_3$ | 138-141 |
| 69 | 3-Cl, 4-$CH_3$ | $CH_3$ | S | $CH_3$ | 121-122 |
| 70 | 4-$(iC_3H_7)_2NCO-$ | $CH_3$ | S | $CH_3$ | 174-176 |
| 71 | 3,4$(Br)_2$ | $CH_3$ | S | $CH_3$ | 104-106 |

(Fortsetzung)

| Verb. Nr. | $R_n$ | $R_1$ | X | $R_2$ | phys. Daten Smp. °C |
|---|---|---|---|---|---|
| 72 | 3-Cl, 4-Br | $CH_3$ | S | $CH_3$ | 115-121 |
| 73 | 3-$CF_3$, 4-Cl | $CH_3$ | S | $CH_3$ | 113-115 |
| 74 | 3-$CH_3$, 4-Br | $CH_3$ | S | $CH_3$ | 110-112 |
| 75 | 3,4-CH=CH-CH=CH- | $CH_3$ | S | $CH_3$ | 105-106 |
| 76 | 4-$CH_3$CONH- | $CH_3$ | S | $CH_3$ | 176-177 |
| 77 | 4-$(CH_3)_2$NCONH-2-$CH_3$ | $CH_3$ | S | $CH_3$ | 206-207 |
| 78 | 4-$CH_3$ | $CH_3$ | S | 4,5-dihydrothien-2-yl | 144-146 |
| 79 | 4-$CH_3$ | $CH_3$ | S | 1-methyl-imidazol-2-yl | 190-192 |
| 80 | 4-$CH_3$ | $CH_3$ | S | 1,2,4-triazol-3-yl | 163-165 |
| 81 | 4-$CH_3$ | $CH_3$ | S | pyrid-2-yl | 171-173 |
| 82 | 4-$CH_3$ | $CH_3$ | S | piperid-2-yl | 144-146 |
| 83 | 4-$CH_3$ | $CH_3$ | S | 4,6-dimethyl-piperid-2-yl | 122-124 |
| 84 | 4-$CH_3$ | $CH_3$ | S | benzoxazol-2-yl | 130-131 |
| 85 | 4-$CH_3$ | $CH_3$ | S | benzthiazol-2-yl | 152-153 |
| 86 | 4-$C_2H_5$ | $CH_3$ | S | $CH_3$ | |
| 87 | 4-$C_2H_5$ | $CH_3$ | O | $CH_3$ | |
| 88 | 4-n $C_4H_9$ | $CH_3$ | O | $CH_3$ | |
| 89 | 4-s $C_4H_9$ | $CH_3$ | S | $CH_3$ | |
| 90 | 4-i. $C_4H_9$ | $CH_3$ | O | $CH_3$ | |
| 91 | 4-t. $C_4H_9$ | $CH_3$ | S | $CH_3$ | |
| 92 | 4-$CH_2$=$CHCH_2$ | $CH_3$ | S | $CH_3$ | |
| 93 | 2-$CH_2$=CH-$CH_2$ | $CH_3$ | S | $CH_3$ | |
| 94 | 4-$C_6H_5$ | $CH_3$ | S | $CH_3$ | |
| 95 | 4-$C_6H_5$-$CH_2$ | $CH_3$ | S | $CH_3$ | |
| 96 | 4-$CF_3$ | $CH_3$ | S | $CH_3$ | |
| 97 | 4-$CH_3$O | $CH_3$ | S | $CH_3$ | |
| 98 | 3-Cl, 4-$CH_3$O | $CH_3$ | S | $CH_3$ | |
| 99 | 2-Cl, 4-$CH_3$O | $CH_3$ | S | $CH_3$ | |

(Fortsetzung)

| Verb. Nr. | $R_n$ | $R_1$ | X | $R_2$ | phys. Daten Smp. °C |
|---|---|---|---|---|---|
| 100 | 4-$CH_2$=$CHCH_2O$ | $CH_3$ | S | $CH_3$ | |
| 101 | 4-$CH\equiv CCH_2O$ | $CH_3$ | S | $CH_3$ | |
| 102 | 4-$CH_2$=CCl-$CH_2O$ | $CH_3$ | S | $CH_3$ | |
| 103 | 2-$CH_2$=CH-$CH_2O$ | $CH_3$ | S | $CH_3$ | |
| 104 | 4-$CH_2$=CH-$CH_2O$ | $CH_3$ | O | $CH_3$ | |
| 105 | 4-(4-Cl-$C_6H_4$)-$CH_2O$ | $CH_3$ | S | $CH_3$ | |
| 106 | 4-(2',4'-$Cl_2$-$C_6H_3$)$CH_2O$ | $CH_3$ | S | $CH_3$ | |
| 107 | 4-$C_6H_5$-$CH_2O$ | $CH_3$ | O | $CH_3$ | |
| 108 | 4-$C_6H_5O$ | $CH_3$ | S | $CH_3$ | |
| 109 | 4-$C_6H_5O$ | $CH_3$ | O | $CH_3$ | |
| 110 | 4-(4'-Cl-$C_6H_4$)O | $CH_3$ | S | $CH_3$ | |
| 111 | 4-(4'-$NO_2C_6H_4$)O | $CH_3$ | S | $CH_3$ | |
| 112 | 4-(4'-$CF_3$-$C_6H_4$)O | $CH_3$ | S | $CH_3$ | |
| 113 | 4-$CH_3O$-$C_2H_4O$ | $CH_3$ | S | $CH_3$ | |
| 114 | 4-$CH_3O$-$C_2H_4O$ | $CH_3$ | O | $CH_3$ | |
| 115 | 4-$CHF_2O$ | $CH_3$ | S | $CH_3$ | |
| 116 | 4-$CF_3O$ | $CH_3$ | S | $CH_3$ | |
| 117 | 4-$CH_3S$ | $CH_3$ | S | $CH_3$ | |
| 118 | 4-$CH_3SO_2$ | $CH_3$ | S | $CH_3$ | |
| 119 | 4-$CH_3OCOCH_2$ | $CH_3$ | S | $CH_3$ | |
| 120 | 4-NC-$CH_2$ | $CH_3$ | S | $CH_3$ | |
| 121 | 4-$(CH_3)_2$-$NCOCH_2$ | $CH_3$ | S | $CH_3$ | |
| 122 | 2-NC-$CH_2$ | $CH_3$ | S | $CH_3$ | |
| 123 | 4-$(CH_3)_2NSO_2$ | $CH_3$ | S | $CH_3$ | |
| 124 | 4-(4'-Cl-$C_6H_4$)S | $CH_3$ | S | $CH_3$ | |
| 125 | 4-(4'-Cl-$C_6H_4$)$SO_2$ | $CH_3$ | S | $CH_3$ | |
| 126 | 4-$CH_3$ | $CH_3$ | S | furan-2-yl | |
| 127 | 4-$CH_3$ | $CH_3$ | S | 4,5-dihydrofuran-2-yl | |
| 128 | 4-Cl | $CH_3$ | S | thien-2-yl | |

(Fortsetzung)

| Verb. Nr. | $R_n$ | $R_1$ | X | $R_2$ | phys. Daten Smp. °C |
|---|---|---|---|---|---|
| 129 | 4-Cl | $CH_3$ | S | 4,5-dihydrothien-2-yl | |
| 130 | 4-Cl | $CH_3$ | S | pyrrol-2-yl | |
| 131 | 4-$CH_3$ | $CH_3$ | S | 1-methylpyrrol-2-yl | |
| 132 | 4-Cl | $CH_3$ | S | pyrid-3-yl | |
| 133 | 4-$CH_3$ | $CH_3$ | S | benzyl | |
| 134 | 4-$CH_3$ | $CH_3$ | S | 4-chlorbenzyl | |
| 135 | 4-Cl | $CH_3$ | S | phenyl | |
| 136 | 4-Cl | $CH_3$ | S | 4-chlorphenyl | |
| 137 | 4-Cl | $CH_3$ | S | $CH_2C\equiv CH$ | |
| 138 | 4-Cl | $CH_2C\equiv CH$ | S | $CH_2C\equiv CH$ | |
| 139 | 4-$CH_3$ | $CH_3$ | S | $CH_2C\equiv CH$ | |
| 140 | 4-$CH_3$ | $CH_2C\equiv CH$ | S | $CH_2C\equiv CH$ | |

Formulierungsbeispiele

Die Verbindungen der Formel I werden im allgemeinen nicht als solche in der Landwirtschaft eingesetzt. Man verwendet gebrauchsfertig formulierte Mittel, welche entweder direkt oder mit Wasser verdünnt eingesetzt werden können.

Beispiel 3 : Stäubemittel

Zur Herstellung eines a) 5 %igen und b) 2 %igen Stäubemittels werden die folgenden Stoffe verwendet :

a) 5 Teile N-(4-Chlorbenzolsulfonyl)-imino-dithiokohlenstoffsäuredimethylester oder einer Mischung davon mit 2-Chlor-2',6'-diäthyl-N-(butoxymethyl)-acetanilid,
95 Teile Talkum,
b) 2 Teile des obigen Wirkstoffes oder einer Mischung,
1 Teil hochdisperse Kieselsäure,
97 Teile Talkum.

Die Wirkstoffe werden mit den Trägerstoffe vermischt und vermahlen und können in dieser Formel zu Anwendung verstäubt werden.

Beispiel 4 : Granulat

Zur Herstellung eines 5 %igen Granulates werden die folgenden Stoffe verwendet :

5 Teile N-(Benzolsulfonyl)-imino-dithiokohlensäure-dimethyl ester oder einer Mischung davon mit 2-Chlor-2',6'-diäthyl-N-(methoxymethyl)-acetanilid,
0,25 Teile epoxidiertes Pflanzenöl,
0,25 Teile Cetylpolyglykoläther,
3,50 Teile Polyäthylenglykol,
91 Teile Kaolin (Korngrösse 0,3-0,8 mm).

Die Aktivsubstanz oder die Mischung wird mit dem Pflanzenöl vermischt und mit 6 Teilen Aceton gelöst, hierauf wird Polyäthylenglykol und Cetylpolyglykoläther zugesetzt. Die so erhaltenene Lösung

wird auf Kaolin aufgesprüht, und anschliessend wird das Aceton im Vakuum verdampft. Ein derartiges Mikrogranulat lässt sich vorteilhaft in Saatfurchen einarbeiten.

Beispiel 5 : Spritzpulver

Zur Herstellung eines a) 70 %igen, b) 40 %igen, c) und d) 25 %igen, e) 10 %igen Spritzpulvers werden folgenden Bestandteile verwendet :

a) 70 Teile N-(4-Chlorbenzolsulfonyl)-imino-thiokohlensäure, O,S-dimethylester oder einer Mischung davon mit 2-Chlor-2',6'-diäthyl-N-(2''-propoxyäthyl)-acetanilid,
5 Teile Natriumdibutylnaphthylsulfonat,
3 Teile Naphthalinsulfonsäure-Phenolsulfonsäuren-Formaldehyd-Kondensat 3 : 2 : 1,
10 Teile Kaolin,
12 Teile Champagne-Kreide,
b) 40 Teile Wirkstoff oder Mischung wie oben,
5 Teile Ligninsulfonsäure-Natriumsalz,
1 Teil Dibutylnaphthalinsulfonsäure-Natriumsalz,
54 Teile Kieselsäure,
c) 25 Teile Wirkstoff oder Mischung wie oben,
4,5 Teile Calcium-Ligninsulfonat,
1,9 Teile Champagne-Kreide/Hydroxyäthylcellulose-Gemisch (1 : 1),
1,5 Teile Natriumdibutylnaphthalinsulfonat,
19,5 Teile Kieselsäure,
19,5 Teile Champagne-Kreide,
28,1 Teile Kaolin,
d) 25 Teile Wirkstoff oder Mischung wie oben,
2,5 Teile Isooctylphenoxypolyoxyäthylenäthanol,
1,7 Teile Champagne-Kreide/Hydroxyäthylcellulose-Gemisch (1 : 1),
8,3 Teile Natriumaluminiumsilikat,
16,5 Teile Kieselgur,
46 Teile Kaolin,
e) 10 Teile Wirkstoff oder Mischung wie oben,
3 Teile Gemisch der Natriumsalze von gesättigten Fettalkoholsulfaten,
5 Teile Naphthalinsulfonsäure/Formaldehyd-Kondensat,
82 Teile Kaolin.

Die Wirkstoffe werden in geeigneten Mischern mit den Zuschlagstoffen innig vermischt und auf entsprechenden Mühlen und Walzen vermahlen. Man erhält Spritzpulver von vorzüglicher Benetzbarkeit und Schwebefähigkeit, die sich mit Wasser zu Suspensionen der gewünschten Konzentration verdünnen und insbesondere zur Blattapplikation verwenden lassen.

Beispiel 6 : Emulgierbare Konzentrate

Zur Herstellung eines 25 %igen emulgierbaren Konzentrates werden folgende Stoffe verwendet :

25 Teile N-(4-Tolylsulfonyl)-imino-dithiokohlensäure-dimethylester oder einer Mischung davon mit 2-Chlor-6'-äthyl-N-(2''-methoxy-1''-methyläthyl)-acet-o-toluidid,
10 Teile eines Alkylarylsulfonat/Fettalkoholpolyglykoläther-Gemisches,
5 Teile Dimethylformamid,
57 Teile Xylol.

Beispiel 7 : Paste

Zur Herstellung einer 45 %igen Paste werden folgende Stoffe verwendet :

a) 45 Teile N-(Benzolsulfonyl)-imino-thiokohlensäure-O,S-dimethylester oder einer Mischung davon mit 2-Chlor-2',6'-diäthyl-N-(methoxymethyl)-acetanilid,
5 Teile Natriumaluminiumsilikat,
14 Teile Cetylpolyäthylenglykoläther mit 8 Mol Aethylenoxid,
1 Teil Oleylpolyäthylenglykoläther mit 5 Mol Aethylenoxid,
2 Teile Spindelöl,
23 Teile Wasser,
10 Teile Polyäthylenglykol,
b) 45 Teile des obigen Wirkstoffes oder der Mischung,
5 Teile Aethylenglykol,

3 Teile Octylphenoxypolyäthylenglykol mit 9-10 Mol Aethylenoxid pro Mol Octylphenol,
3 Teile von einem Gemisch aromatischer Sulfonsäuren, kondensiert mit Formaldehyd als Ammoniumsalz,
1 Teil Siliconöl in Form einer 75 %igen Emulsion,
0,1 Teile einer Mischung von 1-(3-Chlorallyl)-3,5,7-triazo-azonium-adamantanchlorid mit Natriumcarbonat, Chloridwert min. 11,5 %,
0,2 Teile eines biopolymeren Verdickers mit max. 100 Keimen pro Gramm,
42,7 Teile Wasser.

Die Aktivsubstanz wird mit den Zuschlagstoffen in dazu geeigneten Geräten innig vermischt und vermahlen. Man erhält eine Paste, aus der sich durch Verdünnen mit Wasser Suspensionen jeder gewünschten Konzentration herstellen lassen.

Biologisches Beispiel

Die Fähigkeit der Verbindungen der Formel I, Reiskulturen vor der phytotoxischen Wirkung starker Herbizide zu schützen, kann aus dem folgenden Beispiel ersehen werden. In den Versuchsbeschreibungen werden die Verbindungen der Formel I als Antidote (Gegenmittel) bezeichnet. Die relative Schutzwirkung ist in % angegeben. 0 % bedeutet die Wirkung des Herbizides wenn allein appliziert ; 100 % bedeutet das angestrebte normale Wachstum der Kulturpflanze.

Beispiel 8 : Versuch mit Antidote und Herbizid mit in Wasser gesätem Reis.
Applikation der Antidote während der Samenquellung des Reises.

Reissamen werden während 48 Stunden mit Lösungen der als Antidote zu prüfenden Substanz von 100 ppm getränkt. Anschliessend werden die Samen etwa 2 Stunden trocknen gelassen, bis sie nicht mehr kleben. Plastik Töpfe (8 × 8 cm breit und 10 cm hoch) werden bis 2 cm unter den Rand mit sandigem Lehm gefüllt. Die vorgequollenen Samen werden auf der Bodenfläche des Containers gesät und nur ganz schwach gedeckt. Die Erde wird in einem feuchten (nicht sumpfigen) Zustand gehalten. Dann wird das Herbizid in verdünnter Lösung auf die Bodenoberfläche versprüht. Der Wasserstand wird entsprechend dem Wachstum sukzessive erhöht. 21 Tage danach wird die Schutzwirkung des Antidots in Prozent bonitiert. Als Referenzen dienen dabei die mit dem Herbizid allein behandelten Pflanzen (keine Schutzwirkung) sowie die vollständig unbehandelte Kontrolle (100 % Wachstum). Die Ergebnisse sind untenstehend zusammengefasst.

Als Herbizid wird 2-Chlor-2',6'-diäthyl-N-(2"-propyloxyäthyl)-acetanilid (« Pretilachlor ») in einer Aufwandmenge, die 0,25 kg pro Hektar entspricht, eingesetzt.

| Verbindung Nr. | relative Schutzwirkung in % |
|---|---|
| 1 | 38 |
| 3 | 75 |
| 5 | 50 |
| 7 | 63 |
| 8 | 25 |
| 23 | 63 |
| 25 | 12 |
| 35 | 25 |
| 39 | 75 |
| 41 | 25 |
| 45 | 50 |
| 47 | 12.5 |

Beispiel 9 : Versuch mit Antidote und Herbizid in Reis. Applikation von Antidote und Herbizid als Tankmischung im Vorauflaufverfahren.

Reissamen werden während 48 Stunden in Wasser vorgequollen. Plastik-Container (25 cm lang, 17 cm breit und 12 cm hoch) werden mit Erde gefüllt, in die die vorgequollenen Reissamen eingesät werden. Anschliessend wird die als Antidote zu prüfende Substanz zusammen mit dem Herbizid als Tankmischung versprüht. Der Wasserstand wird entsprechend dem Wachstum der Reispflanzen sukzessive erhöht. 18 Tage nach dem Verpflanzen wird die Schutzwirkung des Antidots in Prozent bonitiert. Als Referenzen dienen dabei die mit Herbizid allein behandelten Pflanzen (keine Schutzwirkung) sowie die vollständig unbehandelte Kontrolle (100 % relative Schutzwirkung).

Die Resultate sind untenstehend zusammengefasst.

Herbizid : 2-Chlor-2',6'-diäthyl-N-(2"-propyloxyäthyl)-acetanilid (« Pretilachlor ») Aufwandmenge 0,5 kg/ha

| Verbindung Nr. | Aufwandmenge kg/ha | relative Schutzwirkung % |
|---|---|---|
| 3 | 0,5 | 38 |
| 5 | 0,5 | 50 |
| 7 | 0,5 | 50 |
| 23 | 0,5 | 63 |
| 39 | 0,5 | 75 |
| 45 | 0,5 | 38 |

Beispiel 10 : Versuche mit Antidote und Herbizid in verpflanztem Reis. Applikationsmethode Tankmischung

Reispflanzen werden bis zum 1 1/2-2 Blattstadium in Erde angezogen. Die Pflanzen werden dann büschelweise (immer 3 Pflanzen zusammen) in Container (47 cm lang, 29 cm breit und 24 cm hoch) in sandigen Lehm verpflanzt. Die Bodenoberfläche wird anschliessend mit Wasser von 1,5-2 cm Höhe beschichtet. 2-3 Tage nach dem Verpflanzen wird das Herbizid zusammen mit der als Antidote zu prüfenden Substanz als Tankmischung direkt ins Wasser appliziert. 24 Tage nach dem Verpflanzen wird die Schutzwirkung des Antidots in Prozent bonitiert. Als Referenzen dienen dabei die mit Herbizid allein behandelten Pflanzen, (keine Schutzwirkung) sowie die vollständig unbehandelte Kontrolle (= 100 % Wachstum). Die Resultate sind untenstehend zusammengefasst.

Herbizid : 2-Chlor-2',6'-diäthyl-N-(2"-propyloxyäthyl) acetanilid « Pretilachlor »

| Verbindung Nr. | Aufwandmenge kg/ha | Herbizid Aufwandmenge kg/ha | relative Schutzwirkung % |
|---|---|---|---|
| 3 | 1 | 1 | 12,5 |
| 3 | 0,75 | 0,75 | 12,5 |
| 5 | 1 | 1 | 38 |
| 5 | 0,75 | 0,75 | 25 |
| 7 | 1 | 1 | 25 |
| 7 | 0,75 | 0,75 | 25 |

Beispiel 11 : Versuch mit Antidote und Herbizid mit trocken gesätem Reis. Applikation der Antidote als Saatbeizung.

Reissamen werden mit der als Antidote zu prüfenden Substanz in einen Glasbehälter gemischt. Samen und Produkt werden durch Schütteln Rotation gut zusammengemischt. Dann werden Container (47 cm lang, 29 cm breit und 24 cm hoch) mit sandiger Lehmerde gefüllt und die gebeizten Samen werden eingesät. Nach dem Bedecken des Samens wird das Herbizid in einer verdünnten Lösung auf die Bodenoberfläche versprüht. Etwa 20 Tage nach der Saat (3-Blattstadium der Reispflanzen) wird die Bodenoberfläche mit 4 cm Wasser Höhe beschichtet. 30 Tage nach der Herbizidapplikation wird die Schutzwirkung des Antidots in Prozent bonitiert. Als Referenzen dienen dabei die mit Herbizid allein behandelten Pflanzen (keine Schutzwirkung) sowie die vollständig unbehandelte Kontrolle (100 % Wachstum).

Die Ergebnisse sind wie folgt :

Herbizid : 2-Chlor-2',6'-diäthyl-N-(2"-propyloxyäthyl)-acetanilid, « Pretilachlor »

| Verbindung Nr. | Aufwandmenge g/kg Samen | Herbizid Aufwandmenge kg/ha | relative Schutzwirkung % |
|---|---|---|---|
| 7 | 2 | 1 | 25 |
| 7 | 2 | 2 | 75 |
| 7 | 2 | 3 | 38 |

Herbizid : 5-Aethyl-N,N-hexamethylenthiocarbamat, « Ordram »

| Verbindung Nr. | Aufwandmenge g/kg Samen | Herbizid Aufwandmenge kg/ha | relative Schutzwirkung % |
|---|---|---|---|
| 5 | 2 | 2 | 38 |
| 7 | 2 | 4 | 50 |

Herbizid : 5-4-Chlorbenzyl-N,N-diäthylthiocarbamat « Thiobencarb »

| Verbindung Nr. | Aufwandmenge g/kg Samen | Herbizid Aufwandmenge kg/ha | relative Schutzwirkung % |
|---|---|---|---|
| 7 | 2 | 4 | 25 |

Herbizid : 2-Chlor-6'-äthyl-N-(2"-methoxy-1"-methyläthyl)-acet-o-toluidid, « Metolachlor »

| Verbindung Nr. | Aufwandmenge g/kg Samen | Herbizid Aufwandmenge kg/ha | relative Schutzwirkung % |
|---|---|---|---|
| 3 | 4 | 0,5 | 25 |
| 5 | 2 | 0,25 | 25 |

Beispiel 12 : Versuch mit Antidote und Herbizid in trocken gesätem Reis.
Das Antidote wird als Samenbeize appliziert.

Reissamen der Sorte IR-36 werden mit der als Safener zu prüfenden Substanz in einen Glasbehälter gemischt. Samen und Produkte werden durch Schütteln und Rotation gut zusammengemischt. Anschliessend werden Plastikcontainer (47 cm lang, 29 cm breit und 24 cm hoch) mit sandiger Lehmerde gefüllt und die gebeizten Samen werden eingesät. Nach dem Bedecken des Samens wird das Herbizid auf die Bodenfläche versprüht. 18 Tage nach der Saat wird die Schutzwirkung des Safeners in Prozent bonitiert. Als Referenzen dienen dabei die mit Herbizid allein behandelten Pflanzen (keine Schutzwirkung) sowie die vollständig unbehandelte Kontrolle (100 % Wachstum).

Die Resultate sind wie folgt :

Herbizid : 2-Chlor-2',6'-diäthyl-N-(2"-propyloxyäthyl) acetanilid « Pretilachlor »

| Verbindung Nr. | Aufwandmenge g/kg Samen | Herbizid Aufwandmenge kg/ha | relative Schutzwirkung % |
|---|---|---|---|
| 7 | 2 | 1 | 25 |
| 7 | 2 | 2 | 75 |
| 7 | 2 | 3 | 38 |

Herbizid : S-Aethyl-N,N-hexamethylenthiocarbamat « Ordram »

| Verbindung Nr. | Aufwandmenge g/kg Samen | Herbizid Aufwandmenge kg/ha | relative Schutzwirkung % |
|---|---|---|---|
| 5 | 2 | 2 | 38 |
| 7 | 2 | 4 | 50 |

Herbizid : S-4-Chlorbenzyl-N,N-diäthylthiocarbamat « Thiobencarb »

| Verbindung Nr. | Aufwandmenge g/kg Samen | Herbizid Aufwandmenge kg/ha | relative Schutzwirkung % |
|---|---|---|---|
| 7 | 2 | 4 | 25 |

Herbizid : 2-Chlor-6'-äthyl-N-(2"-methoxy-1"-methyläthyl) acet-o-toluidid « Metolachlor »

| Verbindung Nr. | Aufwandmenge g/kg Samen | Herbizid Aufwandmenge kg/ha | relative Schutzwirkung % |
|---|---|---|---|
| 3 | 4 | 0,25 | 25 |
| 5 | 2 | 0,25 | 25 |

Beispiel 13 : Versuch mit Antidote und Herbizid in trocken gesätem und nachher überflutetem Reis. Applikation von Herbizid und Antidote als Tankmischung im Vorauflauf.

Reissamen der Sorte IR-36 werden in Container (47 cm lang, 29 cm breit und 24 cm hoch) eingesät, bedeckt und leicht festgedrückt. Dann wird die als Antidote zu prüfende Substanz zusammen mit dem Herbizid als Tankmischung versprüht. Etwa 20 Tage nach der Saat (3-Blattstadium der Reispflanzen) wird die Bodenoberfläche mit 4 cm Wasser Höhe beschichtet. 30 Tage nach der Applikation wird die Schutzwirkung des Antidots in Prozent bonitiert. Als Referenzen dienen dabei die mit Herbizid allein behandelten Pflanzen (keine Schutzwirkung) sowie die vollständig unbehandelte Kontrolle (100 % Wachstum).

Das Resultat ist wie folgt :

Herbizid : 2-Chlor-2',6'-diäthyl-N-(2"-propyloxyäthyl)-acetanilid « Pretilachlor »

| Verbindung Nr. | Aufwandmenge kg/ha | Herbizid Aufwandmenge kg/ha | relative Schutzwirkung % |
|---|---|---|---|
| 7 | 3 | 3 | 63 |
| 7 | 2 | 2 | 75 |
| 7 | 1,5 | 1,5 | 63 |

**Patentansprüche**

1. Herbizides Mittel zum Bekämpfen von Unkräutern in Reiskulturen, welches neben einem Chloracetamilid-Herbizid oder einem anderen herbizid wirksamen Stoff ein Gegenmittel zum Schützen der Reiskulturen vor Schäden, die durch die phytotoxische Wirkung des Herbizides entstehen können; enthält, dadurch gekennzeichnet, dass es als Gegenmittel einen N-Sulfonyl-imino-thiokohlensäurediester der Formel I enthält

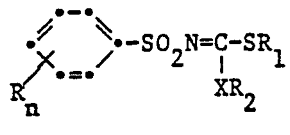

(I)

worin

n 0, 1, 2 oder 3,

R Halogen, Cyan, Nitro, einen Rest —Y—$C_1$-$C_4$ Alkyl; —Y—$C_3$-$C_6$ Alkenyl; —Y—$C_3$-$C_6$-Alkinyl, —Y-Phenyl oder —Y-Aralkyl, welcher unsubstituiert oder durch Halogen, Cyan, Nitro, $C_1$-$C_4$ Alkoxy, $C_1$-$C_4$ Alkoxycarbonyl, Carbamoyl, Sulfamoyl, $C_1$-$C_4$-Mono- oder Dialkylcarbamoyl, $C_1$-$C_4$-Mono- oder Dialkylsulfamoyl substituiert ist, ferner ein Rest —$COOR_3$, —$NR_3R_4$, —$CONR_3R_4$, —$NHCONR_3R_4$ oder —$SO_2NR_3R_4$ oder zwei benachbarte R bilden zusammen eine 3-4 gliedrige gesättigte oder ungesättigte Kohlenwasserstoffkette deren Kettenglieder einmal durch Sauerstoff oder Schwefel und/oder ein- bis dreimal durch Stickstoff ersetzt sein können.

$R_1$ und $R_2$ unabhängig voneinander je einen $C_1$-$C_4$ Alkyl-, $C_3$-$C_6$ Alkenyl-, $C_3$-$C_6$ Alkinyl-, Phenyl- oder Aralkylrest, der unsubstituiert oder durch Halogen, Cyan, Nitro, $C_1$-$C_4$ Alkyl, $C_1$-$C_4$ Halogenalkyl oder $C_1$-$C_4$ Alkoxy substituiert sein kann oder eines von

$R_1$ und $R_2$ kann auch Wasserstoff, oder einen 5-6 gliedrigen, gesättigten oder ungesättigten Heterocyclus bedeuten, der über ein Kohlenstoffatom gebunden ist, 1-3 Heteroatome enthält und auch benzanneliert sein kann und der durch Halogen und/oder Methyl substituiert sein kann,

$R_3$ einen $C_1$-$C_4$ Alkyl-, $C_3$-$C_6$-Alkenyl-, $C_3$-$C_6$ Alkinyl-, $C_3$-$C_6$ Cycloalkylrest, welcher durch Halogen, Cyan, Nitro oder $C_1$-$C_4$ Alkyl substituiert sein kann,

$R_4$ Wasserstoff, dasselbe wie $R_3$, einen Phenyl- oder Aralkylrest, welcher durch Halogen, Cyan, Nitro, $C_1$-$C_4$ Alkyl, $C_1$-$C_4$ Halogenalkyl oder $C_1$-$C_4$ Alkoxy substituiert sein kann oder

$R_3$ und $R_4$ bilden zusammen mit dem Stickstoffatom, an das sie gebunden sind, einen 5-6 gliedrigen, gesättigten oder ungesättigten Heterocyclus, der noch Sauerstoff, Schwefel, Stickstoff oder einen Methyl-imminorest als Ringglied enthalten kann,

X Sauerstoff oder Schwefel und

Y die direkte Bindung oder eine Sauerstoff-, Schwefel-, Sulfinyl-, Sulfonyl-, Imino- oder Methyliminobrücke bedeuten

zusammen mit inerten Hilfstoffen und gegebenenfalls dem Herbizid vor dem Schutz angestrebt wird.

2. Mittel gemäss Anspruch 1 welches als Gegenmittel einen N-Sulfonyl-imino-thiokohlensäure-diester der Formel I enthält, worin X Schwefel bedeutet während R, $R_1$, $R_2$ und n die im Anspruch 1 gegebene Bedeutung haben.

3. Mittel gemäss Anspruch 1, dadurch gekennzeichnet, dass es als Gegenmittel einen N-Sulfonyl-imino-thiokohlensäure-diester der Formel I enthält, worin X Schwefel und n 1 oder 2 bedeuten, während R, $R_1$ und $R_2$ die im Anspruch 1 gegebene Bedeutung haben.

4. Mittel gemäss Anspruch 1, dadurch gekennzeichnet, dass es als Gegenmittel einen N-Sulfonyl-imino-thiokohlensäure-diester der Formel I enthält, worin

n 0, 1, 2 oder 3,

R sich in der para- oder meta-Stellung zur Sulfonylgruppe befindet und Halogen, Cyan, $C_1$-$C_4$ Alkyl, $C_1$-$C_4$ Halogenalkyl, $C_1$-$C_4$ Alkoxy, $C_1$-$C_4$ Halogenalkoxy, $C_1$-$C_4$ Alkylthio, $C_1$-$C_4$ Alkylsulfonyl, Sulfamoyl oder $C_1$-$C_4$ Dialkylsulfamoyl bedeutet,

$R_1$ und $R_2$ unabhängig voneinander je $C_1$-$C_4$ Alkyl, $C_2$-$C_5$ Alkoxyalkyl, $C_1$-$C_4$ Halogenalkyl, $C_3$-$C_6$ Alkenyl, $C_3$-$C_6$ Halogenalkenyl oder $C_3$-$C_6$ Alkinyl und

X Sauerstoff oder Schwefel bedeuten.

5. Mittel gemäss Anspruch 1, dadurch gekennzeichnet, dass es als Gegenmittel einen N-Sulfonyl-imino-thiokohlensäure-diester der Formel I enthält, worin X Schwefel, n 1 oder 2 bedeutet, der Rest R sich in der para- oder meta-Stellung zur Sulfonylgruppe befindet während $R_1$ und $R_2$ die im Anspruch 1 gegebene Bedeutung haben.

6. Mittel gemäss Anspruch 1, dadurch gekennzeichnet, dass es als Gegenmittel einen N-Sulfonyl-imino-thiokohlensäure-diester der Formel I enthält, worin X Schwefel, n 1 oder 2, R Halogen und gegebenenfalls —Y—$C_1$—$C_4$-Alkyl, Y—$C_1$—$C_4$-Halogenalkyl, Cyan oder Nitro, Y die direkte Bindung oder Sauerstoff bedeuten während $R_1$ und $R_2$ die im Anspruch 1 gegebene Bedeutung haben.

7. Mittel gemäss Anspruch 1, dadurch gekennzeichnet, dass es als Gegenmittel einen N-Sulfonyl-imino-thiokohlensäure-diester der Formel I enthält, worin X Schwefel und $R_1$ und $R_2$ je Methyl bedeuten während R und n die im Anspruch 1 gegebene Bedeutung haben.

8. Mittel gemäss Anspruch 1, dadurch gekennzeichnet, dass es als Gegenmittel N-(4-Chlorbenzolsulfonyl)-imino-dithiokohlensäure-dimethylester enthält.

9. Mittel gemäss Anspruch 1, dadurch gekennzeichnet, dass es als Gegenmittel N-(4-Brombenzolsulfonyl)-imino-dithiokohlensäure-dimethylester enthält.

10. Mittel gemäss Anspruch 1, dadurch gekennzeichnet, dass es als Gegenmittel N-(3,4-Dichlorbenzolsulfonyl)-imino-dithiokohlensäure-dimethylester enthält.

11. Verwendung der N-Sulfonyl-imino-thiokohlensäure-diester der Formel I gemäss Anspruch 1 zum Schützen von Reiskulturen vor der Schädigung durch Chloracetanilid-Herbizide oder andere herbizid wirksame Stoffe.

12. Verfahren zum Schützen von Reiskulturen vor Schäden, die bei der Applikation von Herbiziden auftreten, dadurch gekennzeichnet, dass man

a) die Reis-Anbaufläche vor oder während der Applikation des Hervizides oder

b) den Reissamen oder die jungen Reispflänzchen (Stecklinge) selbst mit einer wirksamen Menge eines N-Sulfonyl-imino-thiokohlensäure-diestern der Formel I gemäss Anspruch 1 behandelt.

13. Das durch die N-Sulfonyl-imino-thiokohlensäure-diester der Formel I, Anspruch 1, behandelte Saatgut.

## Claims

1. A herbicidal composition for controlling weeds in rice crops which contains, in addition to a chloroacetanilide herbicide or another herbicide, a safener for protecting said rice crops from the

phytotoxic action of the herbicide, which composition contains as safener an N-sulfonylimino-dithiocarbonate of formula I

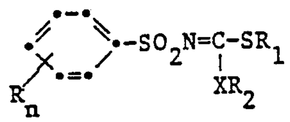
(I)

wherein

n is 0, 1, 2 or 3,

R is halogen, cyano, nitro, a —Y—$C_1$-$C_4$ alkyl radical, —Y—$C_3$-$C_6$ alkenyl, —Y—$C_3$-$C_6$ alkynyl, —Y-phenyl or —Y-aralkyl, each unsubstituted or substituted by halogen, cyano, nitro, $C_1$-$C_4$ alkoxy, $C_1$-$C_4$-alkoxy-carbonyl, carbamoyl, sulfamoyl, $C_1$-$C_4$ mono- or dialkylcarbamoyl, $C_1$-$C_4$ mono- or dialkylsulfamoyl, or is a —$COOR_3$, —$NR_3R_4$, —$CONR_3R_4$, —$NHCONR_3R$ or —$SO_2NR_3R_4$ radical, or two adjacent R substituents together form a 3- or 4-membered, saturated or unsaturated hydrocarbon chain, the chain members of which can be replaced once by oxygen or sulfur and/or once to three times by nitrogen,

$R_1$ and $R_2$ are each independently ot the other a $C_1$-$C_4$ alkyl, $C_3$-$C_6$ alkenyl, $C_3$-$C_6$ alkynyl, phenyl or aralkyl group which is unsubstituted or substituted by halogen, cyano, nitro, $C_1$-$C_4$ alkyl, $C_1$-$C_4$ haloalkyl or $C_1$-$C_4$ alkoxy, or one of

$R_1$ and $R_2$ may also be hydrogen or can form a 5- or 6-membered, saturated or unsaturated heterocycle which is bound through a carbon atom, contains 1-3 hetero atoms and can also be fused with benzene nuclei, and which may be substituted by halogen and/or methyl,

$R_3$ is a $C_1$-$C_4$ alkyl, $C_3$-$C_6$ alkenyl, $C_3$-$C_6$ alkynyl or $C_3$-$C_6$ cycloalkyl group, each of which may be sustituted by halogen, cyano, nitro or $C_1$-$C_4$ alkyl,

$R_4$ is hydrogen, has the same meaning as $R_3$, or is a phenyl or aralkyl group which may be substituted by halogen, cyano, nitro, $C_1$-$C_4$ alkyl, $C_1$-$C_4$ haloalkyl or $C_1$-$C_4$ alkoxy or

$R_3$ and $R_4$ together with the nitrogen atom to which they are attached form a 5- or 6-membered, saturated or unsaturated heterocycle which may also contain oxygen, sulfur, nitrogen or a methylimino group as ring member,

X is oxygen or sulfur, and

Y is a direct bond or an oxygen, sulfur, sulfinyl, sulfonyl, imino or methylimino bridge,

together with inert adjuvants, and optionally the herbicide against which protection is to be afforded.

2. A composition according to claim 1, which contains as safener an N-sulfonyliminodithiocarbonate of the formula I, wherein at least one X is sulfur, and R, $R_1$, $R_2$ and n are as defined in claim 1.

3. A composition according to claim 1, which contains as safener an N-sulfonyliminodithiocarbonate of the formula I, wherein X is sulfur and n is 1 or 2, and R, $R_1$ and $R_2$ are as defined in claim 1.

4. A composition according to claim 1, which contains as safener an N-Sulfonyliminodithiocarbonate of the formula I, wherein

n is 0, 1, 2 or 3,

R is in the para- or meta-position with respect to the sulfonyl group and is halogen, cyano, $C_1$-$C_4$ alkyl, $C_1$-$C_4$ haloalkyl, $C_1$-$C_4$ alkoxy, $C_1$-$C_4$ haloalkoxy, $C_1$-$C_4$ alkylthio, $C_1$-$C_4$ alkylsulfonyl, sulfamoyl or $C_1$-$C_4$ dialkylsulfamoyl,

$R_1$ and $R_2$ are each independently of the other $C_1$-$C_4$ alkyl, $C_2$-$C_5$ alkoxyalkyl, $C_1$-$C_4$ haloalkyl, $C_3$-$C_6$ alkenyl, $C_3$-$C_6$ haloalkenyl or $C_3$-$C_6$ alkynyl, and

X is oxygen or sulfur.

5. A composition according to claim 1, which contains as safener an N-sulfonyliminodithiocarbonate of the formula I, wherein X is sulfur, n is 1 or 2, R is in the para- or meta-position with respect to the sulfonyl group, and $R_1$ and $R_2$ are as defined in claim 1.

6. A composition according to claim 1, which contains as safener an N-sulfonyliminodithiocarbonate of the formula I, wherein X is sulfur, n is 1 or 2, R is halogen and optionally —Y—$C_1$-$C_4$ alkyl, —Y—$C_1$-$C_4$ haloalkyl, cyano or nitro, Y is a direct bond or oxygen, and $R_1$ and $R_2$ are as defined in claim 1.

7. A composition according to claim 1, which contains as safener an N-sulfonyliminodithiocarbonate of the formula I, wherein X is sulfur, and $R_1$ and $R_2$ are each methyl, and R and n are as defined in claim.

8. A composition according to claim 1, which contains as safener dimethyl N-(4-chlorobenzenesulfonyl) iminodithiocarbonate.

9. A composition according to claim 1, which contains as safener dimethyl N-(4-bromobenzenesulfonyl) iminodithiocarbonate.

10. A composition according to claim 1, which contains as safener dimethyl N-(3,4-dichlorobenzenesulfonyl) iminodithiocarbonate.

11. Use of an N-sulfonyliminodithiocarbonate of the formula I according to claim 1, for protecting rice crops from damage caused by chloroacetanilide herbicides or by other herbicidally active substances.

12. A method of protecting rice plants from damage arising from the application of herbicides, which method comprises

a) treating the cultivated area for the rice crops before or during application of the herbicide, or

b) treating the rice seeds or the rice seedlings themselves with an effective amount of an N-sulfonyliminodithiocarbonate of formula I according to claim 1.

13. Seeds treated with an N-sulfonyliminodithiocarbonate of formula I according to claim 1.

**Revendications**

1. Produit herbicide prévu pour combattre les mauvaises herbes dans les cultures de riz, contenant, avec un herbicide du type chloracétanilide ou une autre substance à activité herbicide, un antidote servant à protéger les cultures de riz contre les dommages résultant des effets phytotoxiques de l'herbicide, caractérisé en ce quil contient en tant qu'antidote un diester N-sulfonyl-imino-thiocarbonique de formule I

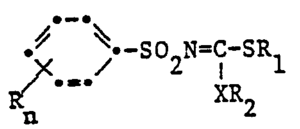

(I)

dans laquelle

n est égal à 0, 1, 2 ou 3,

R représente un halogène, un groupe cyano, nitro, un reste —Y-alkyle en $C_1$-$C_4$, —Y-alcényle en $C_3$-$C_6$, —Y-alcynyle en $C_3$-$C_6$, —Y-phényle ou —Y-aralkyle non substitué ou substitué par des halogènes, des groupes cyano, nitro, alcoxy en $C_1$-$C_4$)-carbonyle, carbamoyle; sulfamoyle, mono- ou di-(alkyle en $C_1$-$C_4$)-carbamoyle, mono- ou di-(alkyle ou $C_1$-$C_4$)-sulfamoyle, ou encore un groupe $—COOR_3$, $—NR_3R_4$, $—CONR_3R_4$, $—NHCONR_3R_4$ ou $—SO_2NR_3R_4$ ou bien deux symboles R voisins représentent ensemble une chaîne hydrocarbonée saturée ou insaturée à 3-4 chaînons dont les chaînons peuvent être remplacés une fois par l'oxygène ou le soufre et/ou 1 à 3 fois par l'azote,

$R_1$ et $R_2$ représentent chacun, indépendamment l'un de l'autre, un groupe alkyle en $C_1$-$C_4$, alcényle en $C_3$-$C_6$, alcynyle en $C_3$-$C_6$, phényle ou aralkyle non substitué ou substitué par des halogènes, des groupes cyano, nitro, alkyle en $C_1$-$C_4$, halogénoalkyle en $C_1$-$C_4$ ou alcoxy en $C_1$-$C_4$ ou bien l'un des symboles $R_1$ et $R_2$ peut également représenter l'hydrogène ou un hétérocycle saturé ou insaturé à 5-6 chaînons relié par l'intermédiaire d'un atome de carbone, contenant 1 à 3 hétéroatomes et qui peut également être condensé avec un noyau benzénique et substitué par des halogènes et/ou des groupes méthyle,

$R_3$ représente un groupe alkyle en $C_1$-$C_4$, alcényle en $C_3$-$C_6$, alcynyle en $C_3$-$C_6$, cycloalkyle en $C_3$-$C_6$ qui peut être substitué par des halogènes, des groupes cyano, nitro ou alkyle en $C_1$-$C_4$,

$R_4$ représente l'hydrogène, ou bien il a les mêmes significations que $R_3$, ou bien il représente un groupe phényle ou aralkyle qui peut être substitué par des halogènes, des groupes cyano, nitro, alkyle en $C_1$-$C_4$, halogénoalkyle en $C_1$-$C_4$ ou alcoxy en $C_1$-$C_4$, ou bien

$R_3$ et $R_4$ forment ensemble et avec l'atome d'azote auquel ils sont reliés un hétérocycle saturé ou insaturé à 5-6 chaînons qui peut encore contenir en tant que chaînon cyclique l'oxygène, le soufre, l'azote ou un groupe méthylimino,

X représente l'oxygène ou le soufre, et

Y représente une liaison directe ou un pont oxygène, soufre, sulfinyle, sulfonyle, imino ou méthylimino,

avec des produits auxiliaires inertes et, le cas échéant, l'herbicide contre lequel on veut protéger.

2. Produit selon la rev. 1, contenant en tant qu'antidote un diester N-sulfonyl-imino-thiocarbonique de formule I dans laquelle X représente le soufre et R, $R_1$, $R_2$ et n ont les significations indiquées dans la rev. 1.

3. Produit selon la rev. 1, caractérisé en ce qu'il contient en tant qu'antidote un diester N-sulfonyl-imino-thiocarbonique de formule I dans laquelle X représente le soufre et n est égal à 1 ou 2, R, $R_1$ et $R_2$ ayant les significations indiquées dans la rev. 1.

4. Produit selon la rev. 1, caractérisé en ce qu'il contient en tant qu'antidote un diester N-sulfonyl-imino-thiocarbonique de formule I dans laquelle n est égal à 0, 1, 2 ou 3,

R se trouve en position para ou méta par rapport au groupe sulfonyle et représente un halogène, un groupe cyano, alkyle en $C_1$-$C_4$, halogénoalkyle en $C_1$-$C_4$, alcoxy en $C_1$-$C_4$, halogénoalcoxy en $C_1$-$C_4$, alkylthio en $C_1$-$C_4$, alkylsulfonyle en $C_1$-$C_4$, sulfamoyle ou di-(alkyle en $C_1$-$C_4$)-sulfamoyle,

$R_1$ et $R_2$ représentent chacun, indépendamment l'un de l'autre, un groupe alkyle en $C_1$-$C_4$, alcoxyalkyle en $C_2$-$C_5$, halogénoalkyle en $C_1$-$C_4$, alcényle en $C_3$-$C_6$, halo ou alcynyle en $C_3$-$C_6$, et

X représente l'oxygène ou le soufre.

5. Produit selon la rev. 1, caractérisé en ce qu'il contient en tant qu'antidote un diester N-sulfonyl-imino-thiocarbonique de formule I dans laquelle X représente le soufre, n est égal à 1 ou 2, le substituant R se trouve en position para ou méta par rapport au groupe sulfonyle et $R_1$ et $R_2$ ont les positions indiquées dans la rev. 1.

6. Produit selon la rev. 1, caractérisé en ce qu'il contient en tant qu'antidote un diester N-sulfonyl-imino-thiocarbonique de formule I dans laquelle X représente le soufre, n est égal à 1 ou 2, R représente un halogène et, le cas échéant, un groupe —Y-alkyle en $C_1$-$C_4$, Y-halogénoalkyle en $C_1$-$C_4$, cyano ou nitro, Y représente une liaison directe ou l'oxygène et $R_1$ et $R_2$ ont les significations indiquées dans la rev. 1.

7. Produit selon la rev. 1, caractérisé en ce qu'il contient en tant qu'antidote un diester N-sulfonyl-imino-thiocarbonique de formule I dans laquelle X représente le soufre et $R_1$ et $R_2$ des groupes méthyle, R et n ayant les significations indiquées dans la rev. 1.

8. Produit selon la rev. 1, caractérisé en ce qu'il contient comme antidote le N-(4-chlorobenzène-sulfonyl)-imino-dithiocarbonate de diméthyle.

9. Produit selon la rev. 1, caractérisé en ce qu'il contient en tant qu'antidote le N-(4-bromobenzène-sulfonyl)-imino-dithiocarbonate de diméthyle.

10. Produit selon la rev. 1, caractérisé en ce qu'il contient en tant qu'antidote le N-(3,4-dichlorobenzène-sulfonyl)-imino-dithiocarbonate de diméthyle.

11. Utilisation des diesters N-sulfonyl-imino-thiocarboniques de formule I selon la rev. 1 pour la protection des cultures de riz contre les dommages provoqués par des herbicides du type chloracétanilides ou d'autres substances possédant une activité herbicide.

12. Procédé pour protéger les cultures de riz contre les dommages résultant de l'application d'herbicides, caractérisé en ce que l'on traite par une quantité efficace d'un diester N-sulfonyl-imino-thiocarbonique de formule I selon la rev. 1

a) l'aire de culture du riz avant ou après l'application de l'herbicide, ou bien

b) les semences de riz ou les jeunes plants de riz (pousses) eux-mêmes.

13. Les semences traitées par les diesters N-sulfonyl-imino-thiocarboniques de formule I, rev. 1.